# EUROPEAN PATENT APPLICATION

(11) **EP 4 660 321 A1**
(43) Date of publication of application: **10.12.2025**
(21) Application number: 24180254.5
(22) Date of filing: 05.06.2024
(51) Int. Cl.: C12Q 1/6883

(54) **GENETIC TEST FOR RESISTANCE TO POST VACCINATION HEPATOPATHY**

(71) Applicant: Hendrix Genetics Research, Technology & Services B.V., 5831CK Boxmeer (NL)
(72) Inventor: Bickhart, Derek, 5831 CK Boxmeer (NL); Machado, Juliana, 5831 CK Boxmeer (NL); Veninga, Gosse, 5831 CK Boxmeer (NL); Ducro-Steverink, Dorothe, 5831 CK Boxmeer (NL)
(74) Representative: Nederlandsch Octrooibureau

(57) **Abstract**

The present invention relates to haplotypes and alleles that are linked to a reduced risk of developing post-vaccination hepatopathy in avian subjects, particularly chicken. Specifically, the invention relates to methods for testing an avian subject for a predisposition for having a reduced risk of developing post-vaccination hepatopathy, which method comprises genotyping the subject for the presence of a haplotype associated with the predisposition. The invention further relates to methods for selective breeding of avian subjects for a predisposition for having a reduced risk of developing post-vaccination hepatopathy using marker-assisted breeding for the presence of the haplotype associated with the predisposition. The invention also relates to isolated nucleic acid molecules or construct comprising the haplotype or a part or variant thereof, and to markers, oligonucleotide probe, primer, arrays and kits for use in the identification of a haplotype.

## Description

### Field of the invention

The present invention relates to the fields of molecular genetics, veterinary medicine, animal husbandry and animal breeding. Specifically, the invention relates to genetic analysis to identifying haplotypes and alleles that are linked to a reduced risk of developing post-vaccination hepatopathy in avian subjects, particularly chicken.

### Background of the invention

We describe a condition of hepatopathy that occurs among Shaver white laying hens (Hendrix Genetics). The condition is manifest as a significant increase in bird mortality following week 38 of development. Post-mortem autopsies reveal significant lesions or plaques on an enlarged liver, which are often accompanied by hemorrhagic enteritis. Some cases indicate swelling of the spleen or obvious epidermal reactions at the injection sites of vaccination. The timing of the manifestation coincides with vaccination or bacterin administration, leading to the hypothesis that the disease is caused (at least in-part) by immune system challenges to the birds. Repeated testing has not confirmed the presence of any endemic viruses in these flocks, which does not support a prognosis of a common disease vector that is causal for the increases in mortality. While, the condition was first reported in Shaver white laying hens product lines, there is evidence that this condition also impacts birds from other genetics companies (see below). Additionally, this disease phenotype is not prevalent in the parental chicken lines used to produce the Shaver white crossbred product for consumers. Outbreaks of this condition in a flock can result in 1% mortality per week after the initial incidence, thereby impacting production substantially.

The disease has similar symptoms to Rift Valley Fever, which is a viral-associated disease that affects livestock in tropical climates. Rift Valley Fever is characterized by liver lesions, internal hemorrhage, and an enlarged spleen (Kainulainen et al., 2014, J. Virol. 88, 3464-3473). The Rift Valley Fever Virus (RVFV) targets subunits of the essential transcription factor, TFIIH, which causes liver damage due to the repression of several key metabolic and repair systems essential to liver function (Rimel and Taatjes, 2018, Protein Sci. Publ. Protein Soc. 27, 1018-1037). Down-regulation of TFIIH has been associated with other liver diseases, such as Hepatitis B, suggesting that it may be a susceptibility factor in animals that have a predilection towards the development of liver abscesses (Jaitovich-Groisman et al., 2001, J. Biol. Chem. 276, 14124-14132).

There is a need in the art to identify the underlying genetic cause of the above-described post-vaccination hepatopathy in chicken so as to develop genetic tests to identify animals with a predisposition for the condition and/or that have a resistance to the condition. It is thus an object of the present invention to provide for methods and means for such genetic tests.

### Summary of the invention

In a first aspect, there is provide a method of testing an avian subject for a predisposition for having a reduced risk of developing post-vaccination hepatopathy. In one embodiment, the method comprises determining in a sample comprising nucleic acids of the subject the presence of a haplotype associated with the predisposition, wherein the haplotype comprises at least one SNP selected from the group consisting of: - A on position 11706079 of chromosome 5 of the galGal4 reference chicken genome assembly; - G on position 11821692 of chromosome 5 of the galGal4 reference chicken genome assembly; - C on position 11903264 of chromosome 5 of the galGal4 reference chicken genome assembly; - G on position 12012528 of chromosome 5 of the galGal4 reference chicken genome assembly; - A on position 12224278 of chromosome 5 of the galGal4 reference chicken genome assembly; - A on position 12365008 of chromosome 5 of the galGal4 reference chicken genome assembly; - A on position 12417674 of chromosome 5 of the galGal4 reference chicken genome assembly; and, - G on position 12442360 of chromosome 5 of the galGal4 reference chicken genome assembly. In one embodiment, the method is a method wherein the presence of the haplotype is determined by detecting in the nucleic acid sample at least one SNP selected from the group consisting of: - A on position 11706079 of chromosome 5 of the galGal4 reference chicken genome assembly; - G on position 11821692 of chromosome 5 of the galGal4 reference chicken genome assembly; - C on position 11903264 of chromosome 5 of the galGal4 reference chicken genome assembly; - G on position 12012528 of chromosome 5 of the galGal4 reference chicken genome assembly; - A on position 12224278 of chromosome 5 of the galGal4 reference chicken genome assembly; - A on position 12365008 of chromosome 5 of the galGal4 reference chicken genome assembly; - A on position 12417674 of chromosome 5 of the galGal4 reference chicken genome assembly; and, - G on position 12442360 of chromosome 5 of the galGal4 reference chicken genome assembly. In one embodiment, the method is a method wherein the avian subject of the genus *Gallus,* preferably of the species *Gallus gallus domesticus.*

In a second aspect, there is provided a method for selective breeding of avian subjects for a predisposition for having a reduced risk of developing post-vaccination hepatopathy, the method comprising: a) determining in a sample comprising nucleic acids of the subject the presence of a haplotype as defined in claim 1, wherein preferably the presence of the haplotype is determined by detecting in the nucleic acid sample at least one SNP selected from the group consisting of: - A on position 11706079 of chromosome 5 of the galGal4 reference chicken genome assembly; - G on position 11821692 of chromosome 5 of the galGal4 reference chicken genome assembly; - C on position 11903264 of chromosome 5 of the galGal4 reference chicken genome assembly; - G on position 12012528 of chromosome 5 ofthe galGal4 reference chicken genome assembly; - A on position 12224278 of chromosome 5 ofthe galGal4 reference chicken genome assembly; - A on position 12365008 of chromosome 5 ofthe galGal4 reference chicken genome assembly; - A on position 12417674 of chromosome 5 of the galGal4 reference chicken genome assembly; and, - G on position 12442360 of chromosome 5 of the galGal4 reference chicken genome assembly, and, b) removing a subject as breeding stock when the haplotype is absent and selecting a subject as breeding stock when the haplotype is present. In one embodiment, the method is a method wherein an avian subject that is selected as breeding stock is homozygous for the haplotype. In one embodiment, the method is a method wherein the avian subject of the genus *Gallus,* preferably of the species *Gallus gallus domesticus.*

In a third aspect, there is provided the use of at least one SNP as defined in the first aspect above, for identification of a haplotype that is indicative of an avian subject subject's predisposition for having a reduced risk of developing post-vaccination hepatopathy, wherein preferably, the avian subject of the genus *Gallus,* preferably of the species *Gallus gallus domesticus.*

In a fourth aspect, there is provided an isolated nucleic acid molecule or construct comprising a haplotype or a part or variant thereof, that is indicative of an avian subject subject's predisposition for having a reduced risk of developing post-vaccination hepatopathy, as comprised in the genome of Shaver White chicken, located on chromosome 5 within the chromosomal region delimited by: - position 11706079 of chromosome 5 of the galGal4 reference chicken genome assembly; and, - position 12442360 of chromosome 5 of the galGal4 reference chicken genome assembly, wherein the nucleic acid molecule or construct, preferably comprises at least one SNP selected from the group consisting of: - A on position 11706079 of chromosome 5 of the galGal4 reference chicken genome assembly; - G on position 11821692 of chromosome 5 of the galGal4 reference chicken genome assembly; - C on position 11903264 of chromosome 5 of the galGal4 reference chicken genome assembly; - G on position 12012528 of chromosome 5 of the galGal4 reference chicken genome assembly; - A on position 12224278 of chromosome 5 of the galGal4 reference chicken genome assembly; - A on position 12365008 of chromosome 5 of the galGal4 reference chicken genome assembly; - A on position 12417674 of chromosome 5 of the galGal4 reference chicken genome assembly; and, - G on position 12442360 of chromosome 5 of the galGal4 reference chicken genome assembly. In one embodiment, the isolated nucleic acid molecule or construct comprises a variant of the haplotype or a part thereof that has at least 60% nucleotide sequence identity with the haplotype as comprised in the genome of Shaver White chicken.

In a fifth aspect, there is provided a marker for identification of a haplotype, wherein the haplotype, when present in the genome of an avian subject, is indicative of the subject's predisposition for having a reduced risk of developing post-vaccination hepatopathy, wherein the marker is a SNP selected from the group consisting of: - A on position 11706079 of chromosome 5 of the galGal4 reference chicken genome assembly; - C on position 11903264 of chromosome 5 of the galGal4 reference chicken genome assembly; - G on position 12012528 of chromosome 5 of the galGal4 reference chicken genome assembly; - A on position 12365008 of chromosome 5 of the galGal4 reference chicken genome assembly; and, - G on position 12442360 of chromosome 5 of the galGal4 reference chicken genome assembly. In one embodiment, the marker is a marker for identification of the haplotype in the genome of an avian subject of the genus *Gallus,* preferably of the species *Gallus gallus domesticus.*

In a sixth aspect, there is provided an oligonucleotide probe or primer comprising a SNP selected from the group consisting of: - A on position 11706079 of chromosome 5 of the galGal4 reference chicken genome assembly; - G on position 11821692 of chromosome 5 of the galGal4 reference chicken genome assembly; - C on position 11903264 of chromosome 5 of the galGal4 reference chicken genome assembly; - G on position 12012528 of chromosome 5 of the galGal4 reference chicken genome assembly; - A on position 12224278 of chromosome 5 of the galGal4 reference chicken genome assembly; - A on position 12365008 of chromosome 5 of the galGal4 reference chicken genome assembly; - A on position 12417674 of chromosome 5 of the galGal4 reference chicken genome assembly; and, - G on position 12442360 of chromosome 5 of the galGal4 reference chicken genome assembly, or an oligonucleotide probe or primer comprising a genomic sequence immediately adjacent of the SNP.

In a seventh aspect there is provided an array comprising at least one nucleic acid probe as defined in the sixth aspect above.

In an eighth aspect there is provided a kit comprising at least one nucleic acid probe or primer as defined in the sixth aspect above, at least one array as defined in the seventh aspect above, and optionally at least another component for detecting in a sample comprising nucleic acids of an avian subject the presence of a haplotype as defined in the first aspect above.

### Description of the invention

### Definitions

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

"A," "an," and "the": these singular form terms include plural referents unless the content clearly dictates otherwise. The indefinite article "a" or "an" thus usually means "at least one". Thus, for example, reference to "a cell" includes a combination of two or more cells, and the like.

"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods. Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth.

"And/or": The term "and/or" refers to a situation wherein one or more of the stated cases may occur, alone or in combination with at least one of the stated cases, up to with all of the stated cases.

"Comprising": this term is construed as being inclusive and open ended, and not exclusive. Specifically, the term and variations thereof mean the specified features, steps or components are included. These terms are not to be interpreted to exclude the presence of other features, steps or components.

"Exemplary": this term means "serving as an example, instance, or illustration," and should not be construed as excluding other configurations disclosed herein.

"Sequence identity" is herein defined as a relationship between two or more amino acid (polypeptide or protein) sequences or two or more nucleic acid (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods. The terms "sequence identity" or "sequence similarity" means that two (poly)peptide or two nucleotide sequences, when optimally aligned, preferably over the entire length (of at least the shortest sequence in the comparison) and maximizing the number of matches and minimizes the number of gaps such as by the programs ClustalW (1.83), GAP or BESTFIT using default parameters, share at least a certain percentage of sequence identity as defined elsewhere herein. GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is BLOSUM62 (Henikoff & Henikoff, 1992, PNAS 89, 915-919). A preferred multiple alignment program for aligning protein sequences of the invention is ClustalW (1.83) using a BLOSUM matrix and default settings (Gap opening penalty:10; Gap extension penalty: 0.05). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are BLOSUM62 for proteins and DNAFull for DNA). When sequences have a substantially different overall lengths, local alignments, such as those using the Smith Waterman algorithm, are preferred. Alternatively, percentage similarity or identity may be determined by searching against public databases, using algorithms such as FASTA, BLAST, etc.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains.

A "nucleic acid" or "polynucleotide" according to the present invention may include any polymer or oligomer of pyrimidine and purine bases, preferably cytosine, thymine, and uracil, and adenine and guanine, respectively (See Albert L. Lehninger, Principles of Biochemistry, at 793-800 (Worth Pub. 1982) which is herein incorporated by reference in its entirety for all purposes). The present invention contemplates any deoxyribonucleotide, ribonucleotide or nucleic acid component, and any chemical variants thereof, such as methylated, hydroxymethylated or glycosylated forms of these bases, and the like. The polymers or oligomers may be heterogeneous or homogenous in composition, and may be isolated from naturally occurring sources or may be artificially or synthetically produced. In addition, the nucleic acids may be DNA (optionally cDNA) or RNA, or a mixture thereof, and may exist permanently or transitionally in single-stranded or double-stranded form, including homoduplex, heteroduplex, and hybrid states. An "isolated nucleic acid" is used to refer to a nucleic acid which is no longer in its natural environment, for example in vitro or in a recombinant bacterial or plant cell. The nucleic acid of the invention may be at least one of a recombinant, synthetic and artificial nucleic acid.

The terms "nucleic acid construct", "nucleic acid vector", "vector" and "expression construct" are used interchangeably herein and is herein defined as a man-made nucleic acid molecule resulting from the use of recombinant DNA technology. The terms "nucleic acid construct" and "nucleic acid vector" therefore does not include naturally occurring nucleic acid molecules although a nucleic acid construct may comprise (parts of) naturally occurring nucleic acid molecules. Vectors can comprise further genetic elements to facilitate their use in molecular cloning, such as e.g. selectable markers, multiple cloning sites and the like.

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3' non-translated sequence (3' end) comprising a polyadenylation site.

The terms "marker" or "molecular marker" can be used interchangeably and refer to an indicator which defines a specific genetic and chromosomal location, i.e. marks a particular position on the chromosome. Well-known examples known to the skilled person include amplified fragment length polymorphism (AFLP) markers, restriction fragment length polymorphism (RFLP) markers, single nucleotide polymorphisms (SNPs), sequence-characterized amplified regions (SCARs), cleaved amplified polymorphic sequence (CAPS) markers or isozyme markers or combinations of the markers described herein. If a molecular marker is tightly linked to a QTL or haplotype it "marks" the DNA on which the QTL or haplotype is found and can therefore be used in a (molecular) marker assays to select for or against the presence of the QTL or haplotype, e.g. in marker assisted breeding/selection (MAS) methods. A "(molecular) marker linked to a QTL or haplotype" or "(molecular) marker for identification of a QTL or haplotype" may thus refer to, for example, SNPs or any other type of marker used in the field, and that is linked to the QTL or haplotype.

The term "haplotype" refers to a combination of alleles or DNA markers on one chromosome. At the DNA level, haplotype refers to a sequence of nucleotides found at two or more polymorphic sites in a locus on a single chromosome. There are multiple haplotypes over a given chromosome region within a breeding population. As used herein, haplotype includes a full-haplotype and/or a sub-haplotype. Full-haplotype is the 5' to 3' sequence of nucleotides found at all polymorphic sites examined in a locus on a single chromosome from a single individual, while sub-haplotype refers to the 5' to 3' sequence of nucleotides seen at a subset of the polymorphic sites examined in a locus on a single chromosome from a single individual. Relatedly, the term "haplotype pair" refers to the two haplotypes found for a locus in a single individual. "Haplotyping" is a term for a process for determining one or more haplotypes in an individual and includes use of family pedigrees, molecular techniques and/or statistical inference.

"Quantitative trait locus," (or "QTL"), as used herein is a genomic sequence that is associated with a particular phenotypic trait. Multiple QTL may be identified for a particular trait, and they are frequently found on different chromosomes. The number of QTLs that associate significantly with a particular phenotypic trait may provide an indication of the genetic architecture of a trait, the number of genes that affect the trait, or the extent of the effect of one or more of those genes. One or more QTL that significantly associates with a trait may be candidate genes underlying that trait, which can be sequenced and identified. The significance of the degree of association of a given QTL with a particular trait can be assessed statistically, e.g. through QTL mapping of the alleles that occur in a locus and the phenotypes that they produce. Statistical analysis is preferred to demonstrate whether an observed association with a trait is significant. The presence of a QTL, and its location identify a particular region of the genome as potentially containing a gene that is associated, directly (e.g., structurally) or indirectly (e.g., regulatory) with the trait being analyzed. The probability of association can be plotted for various markers associated with the trait spaced across a chromosome, or throughout the genome. A QTL may be present in a haplotype that is associated with a phenotype.

As used herein, the term "allele" refers to one or more alternative forms of a particular nucleic acid sequence, where the differences may include, without limitation, one or more single nucleotide polymorphisms (SNPs), an insertion, inversion, or deletion. The sequence may or may not be within a gene, and may be within a coding region or noncoding region of a gene, and may, for example, be within a promoter or regulatory regions, an exon, or an intron of a particular gene. An allele can relate to a particular trait or characteristic. An allele may be a naturally occurring allele or a mutant allele. In a diploid cell of an organism, alleles of a given gene are located at a specific location, or locus (loci plural) on a chromosome. One allele is present on each chromosome of the pair of homologous chromosomes. A diploid species may comprise identical alleles of the nucleic acid sequence (homozygous) or two different alleles (heterozygous).

Thus, as used herein, the term "heterozygous" refers to a genetic condition existing when two different alleles reside at a specific locus, for example a locus having alleles A/B, wherein A and B are positioned individually on either of the two homologous chromosomes. Conversely, as used herein, the term "homozygous" refers to a genetic condition existing when two identical alleles reside at a specific locus, for example a locus having alleles A/A, positioned individually on each of the two homologous chromosomes.

"Expression of a gene" refers to the process wherein a DNA region which is operably linked to appropriate regulatory regions, particularly a promoter, is transcribed into an RNA, which is biologically active, e.g. a regulatory non-coding RNA or an RNA which is capable of being translated into a biologically active protein or peptide. Expression in relation to a protein or peptide is to be understood herein as the process of gene expression resulting in production of said protein or peptide.

The term "operably linked" refers to a linkage of polynucleotide elements in a functional relationship. A nucleic acid region is "operably linked" when it is placed into a functional relationship with another nucleic acid region. For instance, a promoter, or rather a transcription regulatory sequence, is operably linked to a coding sequence if it affects the transcription of the coding sequence. Operably linked may mean that the DNA sequences being linked are contiguous.

"Promoter" refers to a nucleic acid fragment that functions to control the transcription of one or more nucleic acids. A promoter fragment is preferably located upstream (5') with respect to the direction of transcription of the transcription initiation site of the gene, and is structurally identified by the presence of a binding site for DNA-dependent RNA polymerase, transcription initiation site(s) and can further comprise any other DNA sequences, including, but not limited to transcription factor binding sites, repressor and activator protein binding sites, and any other sequences of nucleotides known to one of skill in the art to act directly or indirectly to regulate the amount of transcription from the promoter.

Optionally, the term "promoter" may also include the 5' UTR region (5' Untranslated Region) (e.g. the promoter may herein include one or more parts upstream of the translation initiation codon of transcribed region, as this region may have a role in regulating transcription and/or translation). A "constitutive" promoter is a promoter that is active in most tissues under most physiological and developmental conditions. An "inducible" promoter is a promoter that is physiologically (e.g. by external application of certain compounds) or developmentally regulated. A "tissue specific" promoter is only active in specific types of tissues or cells.

A "3' UTR" or "3' non-translated sequence" (also often referred to as 3' untranslated region, or 3'end) refers to the nucleic acid sequence found downstream of the coding sequence of a gene, which comprises for example a transcription termination site and (in most, but not all eukaryotic mRNAs) a polyadenylation signal (such as e.g. AAUAAA or variants thereof). After termination of transcription, the mRNA transcript may be cleaved downstream of the polyadenylation signal and a poly(A) tail may be added, which is involved in the transport of the mRNA to the cytoplasm (where translation takes place).

The term "cDNA" means complementary DNA. Complementary DNA is made by reverse transcribing RNA into a complementary DNA sequence. cDNA sequences thus correspond to RNA sequences that are expressed from genes. As mRNA sequences when expressed from the genome can undergo splicing, i.e. introns are spliced out of the mRNA and exons are joined together, before being translated in the cytoplasm into proteins, it is understood that expression of a cDNA means expression of the mRNA that encodes for the cDNA. The cDNA sequence thus may not be identical to the genomic DNA sequence to which it corresponds as cDNA may comprise only the complete open reading frame, consisting of the joined exons, for a protein, whereas the genomic DNA may comprise exons interspersed by intron sequences. Impairment of expression a protein by genetic modification of a gene encoding the protein may thus not only relate to modifying the sequences encoding the protein, but may also involve mutating intronic sequences of the genomic DNA and/or other gene regulatory sequences of that gene, as long as it results in the impairment of gene expression.

The terms "protein" or "polypeptide" are used interchangeably and refer to molecules consisting of a chain of amino acids, without reference to a specific mode of action, size, 3 dimensional structure or origin. A "fragment" or "portion" of a protein may thus still be referred to as a "protein". An "isolated protein" is used to refer to a protein which is no longer in its natural environment, for example in vitro or in a recombinant bacterial or plant cell. The protein of the invention may be at least one of a recombinant, synthetic and artificial protein.

The term "predisposition" refers to a genetic precondition to have a reduced risk of developing post-vaccination hepatopathy, or to pass on to the offspring specific alleles or variants of a gene responsible for developing such a phenotype.

As used throughout the present invention the term "sample" relates to a specimen taken from egg, feather, blood, semen, skin, nail, comb or tissues obtainable from liver or kidney. The sample or biological sample may contain (or be derived from) a "bodily fluid".

The terms "subject" or "individual" are used interchangeably herein to refer to a vertebrate, preferably an avian, more preferably a domestic fowl species, most preferably a chicken. The term "avian" includes for example chicken, turkey, duck, goose, quail, pigeon, pheasants, parrots, finches, hawks, crows and ratites including ostrich, emu and cassowary.

### Detailed description of the invention

The present invention is directed to the surprising discovery of a hitherto unknown genetic region linked to a predisposition for having a reduced risk of developing post-vaccination hepatopathy. The current inventors identified a haplotype in the Shaver White^{™} breed (Hendrix Genetics) that is linked to the reduced risk of developing post-vaccination hepatopathy. Evidence was obtained that the post-vaccination hepatopathy and the protective haplotype are not exclusive to Hendrix Genetics-specific lines but are also prevalent in competitor's line, indicating a broader relevance of the protective haplotype.

In a first aspect, there is therefore provided method of testing an avian subject for a predisposition for having a reduced risk of developing post-vaccination hepatopathy. In one embodiment, the method comprises determining in a sample comprising nucleic acids of the subject the presence of a haplotype associated with the predisposition for having a reduced risk of developing post-vaccination hepatopathy, which haplotype is herein referred to as the "protective haplotype".

In one embodiment, the protective haplotype comprises at least one SNP selected from the group consisting of:
- A on position 11706079 of chromosome 5 of the galGal4 reference chicken genome assembly;
- G on position 11821692 of chromosome 5 of the galGal4 reference chicken genome assembly;
- C on position 11903264 of chromosome 5 of the galGal4 reference chicken genome assembly;
- G on position 12012528 of chromosome 5 of the galGal4 reference chicken genome assembly;
- A on position 12224278 of chromosome 5 of the galGal4 reference chicken genome assembly;
- A on position 12365008 of chromosome 5 of the galGal4 reference chicken genome assembly;
- A on position 12417674 of chromosome 5 of the galGal4 reference chicken genome assembly; and,
- G on position 12442360 of chromosome 5 of the galGal4 reference chicken genome assembly.
The genomic coordinates as used herein are based on the sequence of the galGal4 reference genome assembly (https://www.ncbi.nlm.nih.gov/assembly/GCF 000002315.3/). These SNPs are further referred to herein by their nucleotide and genomic position only. In one embodiment, the protective haplotype comprises at least two, three, four, five, six, seven or eight SNPs selected from the group consisting of: A on position 11706079; G on position 11821692; C on position 11903264; G on position 12012528; A on position 12224278; A on position 12365008; A on position 12417674; and, G on position 12442360. In one embodiment, the protective haplotype comprises at least the SNPs: A on position 11706079; G on position 11821692; C on position 11903264; and, G on position 12012528. In one embodiment, the protective haplotype comprises at least the SNPs: G on position 11821692; C on position 11903264; and, G on position 12012528. In one embodiment, the protective haplotype comprises at least the SNPs: C on position 11903264; and, G on position 12012528. In a preferred embodiment, the protective haplotype comprises at least the SNP: G on position 12012528.

Thus, in one embodiment, there is provided a method of testing an avian subject for its predisposition for having a reduced risk of developing post-vaccination hepatopathy, the method comprising determining in a sample comprising nucleic acids of the subject the presence of the protective haplotype by detecting in the nucleic acid sample at least one SNP selected from the group consisting of: A on position 11706079; G on position 11821692; C on position 11903264; G on position 12012528; A on position 12224278; A on position 12365008; A on position 12417674; and, G on position 12442360.

In one embodiment, there is provided a method of testing an avian subject for its predisposition for having a reduced risk of developing post-vaccination hepatopathy, the method comprising determining in a sample comprising nucleic acids of the subject the presence of the protective haplotype by detecting in the nucleic acid sample at least two, three, four, five, six, seven or eight SNPs selected from the group consisting of: A on position 11706079; G on position 11821692; C on position 11903264; G on position 12012528; A on position 12224278; A on position 12365008; A on position 12417674; and, G on position 12442360.

In one embodiment, there is provided a method of testing an avian subject for its predisposition for having a reduced risk of developing post-vaccination hepatopathy, the method comprising determining in a sample comprising nucleic acids of the subject the presence of the protective haplotype by detecting in the nucleic acid sample at least the SNPs: A on position 11706079; G on position 11821692; C on position 11903264; and, G on position 12012528; preferably at least the SNPs: G on position 11821692; C on position 11903264; and, G on position 12012528; more preferably at least the SNPs: C on position 11903264; and, G on position 12012528; and most preferably at least the SNP: G on position 12012528.

In one embodiment of the method, the avian subject is of the genus *Gallus.* In a preferred embodiment of the method, the avian subject is of the species *Gallus gallus domesticus.*

In the methods provided herein, the presence of the protective haplotype in the genome of an avian subject can be determined by genotyping the avian subject. Genotyping an avian subject for determining the presence, absence and/or zygosity of the protective haplotype can be performed using any DNA detection method known in the art. Genotyping can for example be performed by sequencing, amplification (polymerase chain) reactions and/or hybridization-based assays.

In one embodiment, the methods described herein include sequencing at least part of a genome of one or more cells from the subject. Sequencing can be, for example, whole genome sequencing. In one embodiment, the method involves high-throughput and/or targeted nucleic acid profiling (for example, sequencing, quantitative reverse transcription polymerase chain reaction, and the like).

In one embodiment, sequencing comprises high-throughput (formerly "next-generation") technologies to generate sequencing reads. In DNA sequencing, a read is an inferred sequence of base pairs (or base pair probabilities) corresponding to all or part of a single DNA fragment. A typical sequencing experiment involves fragmentation of the genome into millions of molecules or generating complementary DNA (cDNA) fragments, which are size-selected and ligated to adapters. The set of fragments is referred to as a sequencing library, which is sequenced to produce a set of reads. Methods for constructing sequencing libraries are known in the art (see, e.g., Head et al., Library construction for next-generation sequencing: Overviews and challenges. Biotechniques. 2014; 56(2): 61-77). A "library" or "fragment library" may be a collection of nucleic acid molecules derived from one or more nucleic acid samples, in which fragments of nucleic acid have been modified, generally by incorporating terminal adapter sequences comprising one or more primer binding sites and identifiable sequence tags. In certain embodiments, the library members (e.g., genomic DNA, cDNA) may include sequencing adaptors that are compatible with use in, e.g., Illumina's reversible terminator method, long read nanopore sequencing, Roche's pyrosequencing method (454), Life Technologies' sequencing by ligation (the SOLiD platform) or Life Technologies' Ion Torrent platform. Examples of such methods are described in the following references: Margulies et al (Nature 2005 437: 376-80); Schneider and Dekker (Nat Biotechnol. 2012 Apr. 10; 30(4):326-8); Ronaghi et al (Analytical Biochemistry 1996 242: 84-9); Shendure et al (Science 2005 309: 1728-32); Imelfort et al (Brief Bioinform. 2009 10:609-18); Fox et al (Methods Mol. Biol. 2009; 553:79-108); Appleby et al (Methods Mol. Biol. 2009; 513:19-39); and Morozova et al (Genomics. 2008 92:255-64), which are incorporated by reference for the general descriptions of the methods and the particular steps of the methods, including all starting products, reagents, and final products for each of the steps.

In one embodiment, the methods described herein includes whole genome sequencing. Whole genome sequencing (also known as WGS, full genome sequencing, complete genome sequencing, or entire genome sequencing) is the process of determining the complete DNA sequence of an organism's genome at a single time. This entails sequencing all of an organism's chromosomal DNA as well as DNA contained in the mitochondria. "Whole genome amplification" ("WGA") refers to any amplification method that aims to produce an amplification product that is representative of the genome from which it was amplified. Non-limiting WGA methods include Primer extension PCR (PEP) and improved PEP (I-PEP), Degenerated oligonucleotide primed PCR (DOP-PCR), Ligation-mediated PCR (LMP), T7-based linear amplification of DNA (TLAD), and Multiple displacement amplification (MDA).

In one embodiment, targeted sequencing is used in the methods described herein (see, e.g., Mantere et al., PLoS Genet 12 e1005816 2016; and Carneiro et al. BMC Genomics, 2012 13:375). Targeted gene sequencing panels are useful tools for analyzing specific mutations in a given sample. Focused panels contain a select set of genes or gene regions that have known or suspected associations with the disease or phenotype under study. In one embodiment, targeted sequencing is used to detect the presence or absence of mutations, e.g. SNPs associated with a disease in a subject in need thereof. Targeted sequencing can increase the cost-effectiveness of variant discovery and detection.

Genotyping for determining the presence or absence and/or zygosity of the protective haplotype can also be performed through hybridization-based methods, including dynamic allele-specific hybridization (DASH), molecular beacons, and SNP microarrays, enzyme-based methods including RFLP, PCR-based, e.g., allelic-specific polymerase chain reaction (AS-PCR), polymerase chain reaction-restriction fragment length polymorphism (PCR-RFLP), multiplex PCR real-time invader assay (mPCR-RETINA), (amplification refractory mutation system (ARMS), Flap endonuclease, primer extension, 5' nuclease, e.g., Taqman or 5' nuclease allelic discrimination assay, and oligonucleotide ligation assay, and methods such as single strand conformation polymorphism, temperature gradient gel electrophoresis, denaturing high performance liquid chromatography, high-resolution melting of the entire amplicon, use of DNA mismatch-binding proteins, SNPlex, and Surveyor nuclease assay.

In one embodiment, a nucleic acid sequence(s) comprising one ore SNPs of the above defined haplotype associated with a predisposition for having a reduced risk of developing post-vaccination hepatopathy, can be incorporated into a genotyping platform, comprising a panel or nucleic acid target array. As used herein, "panel" and "array" are used interchangeably and refer to a solid-state substrate, typically a glass, plastic or silicon slide, used to assay and detect single nucleotide polymorphisms from DNA samples in parallel, and genotypic data is output. Numerous panel/array platforms are commercially available and are well known in the art. By way of example, array genotyping platforms directed to cattle include, but are not limited to Neogen's BOVUHDV03 and GeneSeek Genomic Profiler (GGP) Bovine 150K; Illumina's bovine array beadchips, such as BovineSNP50 DNA Analysis BeadChip and BovineHD DNA Analysis Kit; and ThermoFisher's Axiom Bovine Genotyping 100K Array and Axiom Genome-Wide BOS 1 Bovine Array, Affymetrix platforms, among others. In some embodiments, nucleic acid sequence(s) comprising an SNP of the protective haplotype can be incorporated into such commercially available genotyping platforms. Alternatively, nucleic acid sequence(s) comprising an SNP of the protective haplotype can be incorporated into a custom designed array designed to assay specific pools of SNPs, some of which SNPs may overlap with a commercially available platform. Incorporation of nucleic acid sequence(s) comprising one or more SNPs of the protective haplotype depends on the chemistry of the array platform, but follows the principles of the array technology. For example, nucleic acid sequence(s) comprising an SNP of the protective haplotype SNPs are used to design one or more complementary probe sequences, wherein the probe is affixed to the array.

By way of example, these array platforms represent a subset of a larger number of commercially available array platforms varying in SNP content, density (number), chemistry, throughput (number of samples that can be assayed simultaneously), or other properties. However, arrays generally rely on the hybridization of fragmented single-stranded sample DNA to short single-stranded probes, also known as oligonucleotides, which are typically 25-50 bp long. The probes are typically attached, printed, or synthesized directly onto the array surface in a grid-like fashion onto a two-dimensional solid-state surface, such as glass, plastic or silicon. The probe sequences are designed using apriori DNA sequence knowledge, which may have been collected from a panel of individuals representing genotypic diversity for that species, to artificially synthesize sequences that are complementary to the sample DNA fragment sequences. Hybridization depends on the binding of nucleotide bases with their complementary bases, where the probe and sample DNA are complementary in sequence, and thus hybridize. Even strands of DNA having less than a perfect match can hybridize. Typically, the DNA sample sequence matches the probe at 100%, in some embodiments, 99%, 98%, 97%, 96%, 95%, etc. down to 80%.

Array platforms typically utilize fluorescent dye to generate signals that are created when the dye is excited by a laser and the emission spectra detected by a camera, whereby the signals report specific nucleotide identities at the assayed basepair position within the probe sequence. The resulting raw images are normalized to subtract background signals and converted into genotypic data with confidence scores using computational algorithms (LaFramboise, Thomas, 2009). Variations of array technology exist, and the details provided herein are included by means of example but not by way of limitation.

In a second aspect, there is provide a method for selective breeding of avian subjects for a predisposition for having a reduced risk of developing post-vaccination hepatopathy. In one embodiment, the method comprises a) determining in a sample comprising nucleic acids of an avian subject the absence or presence of the protective haplotype as defined above; and b) removing a subject as breeding stock when the haplotype is absent and selecting a subject as breeding stock when the haplotype is present. The method for selective breeding is thus a marker-assisted breeding wherein the markers can be any of the SNPs defined above that can be determined by aby of the genotyping methods described above. In one embodiment, the subjected selected as breeding stock is a subject that is homozygous for the protective haplotype. In one embodiment of the method for selective breeding, the avian subject is of the genus *Gallus.* In a preferred embodiment of the method for selective breeding, the avian subject is of the species *Gallus gallus domesticus.*

In a third aspect, there is provided a use of at least one SNP selected from the group consisting of: A on position 11706079; G on position 11821692; C on position 11903264; G on position 12012528; A on position 12224278; A on position 12365008; A on position 12417674; and, G on position 12442360, for identification of a haplotype that is indicative of an avian subject subject's predisposition for having a reduced risk of developing post-vaccination hepatopathy. In one embodiment, there is provided a use of at least two, three, four, five, six, seven or eight SNPs selected from the group consisting of: A on position 11706079; G on position 11821692; C on position 11903264; G on position 12012528; A on position 12224278; A on position 12365008; A on position 12417674; and, G on position 12442360, for identification of a haplotype that is indicative of an avian subject subject's predisposition for having a reduced risk of developing post-vaccination hepatopathy. In one embodiment, there is provided a use of at least the SNPs: A on position 11706079; G on position 11821692; C on position 11903264; and, G on position 12012528; preferably at least the SNPs: G on position 11821692; C on position 11903264; and, G on position 12012528; more preferably at least the SNPs: C on position 11903264; and, G on position 12012528; and most preferably at least the SNP: G on position 12012528, for identification of a haplotype that is indicative of an avian subject subject's predisposition for having a reduced risk of developing post-vaccination hepatopathy.

In a fourth aspect, there is provided a nucleic acid molecule or construct comprising a haplotype or a part or variant thereof, that is indicative of an avian subject subject's predisposition for having a reduced risk of developing post-vaccination hepatopathy, as comprised in the genome of Shaver White chicken, located on chromosome 5 within the chromosomal region delimited by: position 11706079 of chromosome 5 of the galGal4 reference chicken genome assembly, and position 12442360 of chromosome 5 of the galGal4 reference chicken genome assembly. In one embodiment, the nucleic acid molecule or construct is an isolated nucleic acid molecule or construct.

In one embodiment, the nucleic acid molecule or construct is delimited at the 5'-end by a SNP selected from the group consisting of: A on position 11706079; G on position 11821692; C on position 11903264; G on position 12012528; A on position 12224278; A on position 12365008; and, A on position 12417674, and delimited at the 3'-end by a SNP selected from the group consisting of: G on position 11821692; C on position 11903264; G on position 12012528; A on position 12224278; A on position 12365008; A on position 12417674; and, G on position 12442360.

In one embodiment, the nucleic acid molecule or construct comprises at least one SNP selected from the group consisting of: at least one SNP selected from the group consisting of: A on position 11706079; G on position 11821692; C on position 11903264; G on position 12012528; A on position 12224278; A on position 12365008; A on position 12417674; and, G on position 12442360. In one embodiment, the nucleic acid molecule or construct comprises at least two, three, four, five, six, seven or eight SNPs selected from the group consisting of: A on position 11706079; G on position 11821692; C on position 11903264; G on position 12012528; A on position 12224278; A on position 12365008; A on position 12417674; and, G on position 12442360. In one embodiment, the nucleic acid molecule or construct comprises at least the SNPs: A on position 11706079; G on position 11821692; C on position 11903264; and, G on position 12012528; preferably at least the SNPs: G on position 11821692; C on position 11903264; and, G on position 12012528; more preferably at least the SNPs: C on position 11903264; and, G on position 12012528; and most preferably at least the SNP: G on position 12012528.

In one embodiment, the nucleic acid molecule or construct comprises a variant of the haplotype or a part thereof that has, with increasing preference, at least 60%, at least 65%, at least 70%, at least 75%, at least 80%, at least 85%, at least 90%, at least 95%, at least 98% or at least 99% nucleotide sequence identity with the above defined haplotype as comprised in the genome of Shaver White chicken.

In a fifth aspect, there is provided marker for identification of a haplotype, wherein the haplotype, when present in the genome of an avian subject, is indicative of the subject's predisposition for having a reduced risk of developing post-vaccination hepatopathy. In one embodiment, the markers is a SNP selected from the group consisting of: A on position 11706079; C on position 11903264; G on position 12012528; A on position 12365008; and, G on position 12442360. In one embodiment, the marker is for identification of the haplotype in the genome of an avian subject that is of the genus *Gallus,* preferably the avian subject is of the species *Gallus gallus domesticus.*

In a sixth aspect, there is provided an oligonucleotide probe or primer for detection of the protective haplotype described herein in any of the genotyping methods described herein. In one embodiment, oligonucleotide probe or primer comprises a SNP that is comprised in the protective haplotype, i.e. the effect allele. In another embodiment, oligonucleotide probe or primer comprises the SNP of the corresponding other allele. Thus, in one embodiment, an oligonucleotide probe or primer for detection of the protective haplotype described herein comprising a SNP selected from the group consisting of: A on position 11706079; C on position 11706079; C on position 11903264; A on position 11903264; G on position 12012528; A on position 12012528; A on position 12365008; G on position 12365008; G on position 12442360; and, A on position 12442360, or the oligonucleotide probe or primer comprises a genomic sequence immediately adjacent of the SNP.

In one embodiment, the oligonucleotide probe or primer comprises a non-naturally occurring sequence, such as a sequence for barcoding or indexing, can which aid in the detection. In one embodiment, the oligonucleotide probe or primer comprises a sequence that is complementary to the genomic sequence that comprises or sits immediately adjacent of the SNP. In one embodiment, the oligonucleotide probe or primer may contain at least 10, 15, 20 or 30 nucleotides and its length may be shorter than 400, 300, 200 or 100 nucleotides.

In a seventh aspect, there is provided an array comprising at least one oligonucleotide probe as described above. The array can be a nucleic acid target array for genotyping as described above.

In an eighth aspect, there is provided a kit for genotyping (and/or haplotyping) a sample from an avian subject, the kit comprising in a container one or more nucleic acid molecules (e.g. an oligonucleotide probe or primer as described above) designed for detecting the one or more polymorphisms associated with the protective haplotype, and optionally at least another component for carrying out such detection. In one embodiment, a kit comprises at least two of the oligonucleotides probe or primer described above packaged in the same or separate containers. The kit may also contain other components such as hybridization buffer (where the oligonucleotides are to be used as a probe) packaged in a separate container. Additionally, or alternatively, where the oligonucleotides are to be used to amplify a target region, the kit may contain, preferably packaged in separate containers, a polymerase and a reaction buffer optimized for primer extension mediated by the polymerase, such as PCR.

The present invention has been described above with reference to a number of exemplary embodiments as shown in the drawings. Modifications and alternative implementations of some parts or elements are possible, and are included in the scope of protection as defined in the appended claims.

### Description of the figures

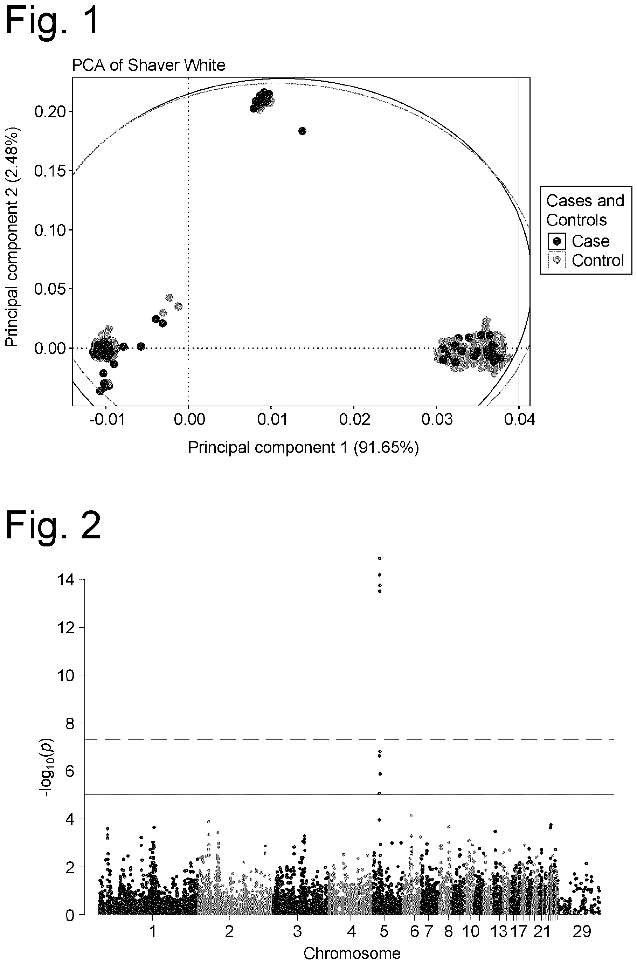

Figure 1. Principal Component analysis to visualize differences in samples (dots) in all flocks. The proportion of the distance explained by the top two Principal components is shown in parentheses on each respective axis. Distance between points indicates the similarity of samples, where closer samples are more similar to each other. The bottom left cluster of samples represents the likely true positive shaver white samples that were correctly selected by veterinarians/staff. Clusters in the top middle and bottom right indicate birds that may have been accidentally sampled from a competitor's line; hence the substantial genetic differences between their groups and the HG lines.
Figure 2. A Manhattan plot showing marker associations with the disease phenotype. The red line indicates genome-wide significance of the association with the disease.

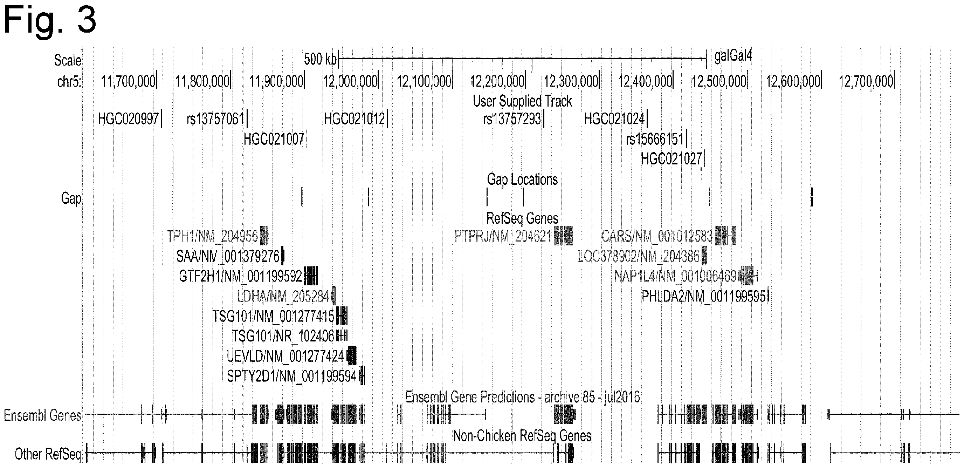

Figure 3. UCSC genome browser screenshot showing the region with significant association to the disease. Tracks identifying the locations of candidate genes are displayed on horizontally descending segments in the following order: 1) Positions of the markers that constitute the discovered haplotype, 2) GalGal4 chicken assembly gap regions, 3) Refseq annotated genes (blue), 4) Genbank annotated genes (black), 5) other Refseq annotated genes derived from orthogonal evidence (ie, RNA sequencing).

### Examples

### Example 1

### 1.1 Description of the experimental design

A case-control experimental design was chosen as the disease phenotype was believed to always cause the mortality of the affected individual. To provide sufficient statistical power, Hendrix Genetics (HG) staff veterinarians were asked to sample 1000+ cases and 1000+ controls from affected flocks. Several privately owned flocks were sampled across the continental US and EU. Since these private flocks were HG customers, they may have contained mixtures of birds from competing genetics companies (ie. chicken lines that are not HG lines). Cases were defined as birds that had all previously described symptoms observed during a postmortem autopsy, and samples of their combs were collected immediately after the autopsy. Control samples were defined as birds that survived the entire trial period and did not manifest any symptoms consistent with the specific hepatopathy phenotype. This meant that there was often a significant delay in the collection of control samples from affected flocks. All collected samples were prepared for DNA extraction at the HG laboratory in Ploufragan, France using a membrane-based DNA extraction kit developed by Macheray (France). All samples passed our minimal quality standards (A260/280 ratios > 1.6; minimum concentration: 50 ng/ul).

### 1.2 Genotyping and association analysis

Sample DNA was sent to Neogen (Lincoln, NE, USA) for genotyping on the HG proprietary Chicken Infinium SNP chip. This chip consists of 60,000 commercial and proprietary markers that were custom selected to maximize the determination of genetic variance within HG laying hen lines. This means that the markers are not always suitable for determining the precise genetic relationships of non-HG lines; however, they can be useful to identify substantial differences in genetic content within the species as a whole. The marker genomic coordinates were based on the galGal6a reference genome assembly. Genotypes were received as Illumina final reports, and were converted to a series of file formats that are amendable to analysis using the Plink line of software tools.

Initial tests focused on pruning the list of samples and markers for suitability in a Genome-Wide Association Study (GWAS). Poorly performing SNP markers were filtered using standard practices found in the scientific literature. The first test was to identify highly divergent samples that would provide more noise in the GWAS and would obscure any true positive signal. In order to do this, a Principal Component Analysis (PCA) was conducted using a filtered set of SNP markers (see Figure 1). Three distinct clusters of samples were identified which indicated the contamination of the sample pool with distantly related individuals. Discussions with veterinary staff and other involved geneticists indicated that the samples that clustered separately from the main group may have consisted of birds from a competitor's line that were housed in the same hatcheries as the Shaver White birds. The external appearance of the HG line and the competitor's line were quite similar, so this mistake was to be expected in these flocks. It was also clear that some cases were identified within the potential competitor cluster which means that this disease is not exclusive to HG specific lines. In order to reduce the potential background noise for a GWAS, samples within the divergent clusters (top middle; bottom right) were removed from subsequent analysis.

The GWAS was conducted using the Plink software platform using default linear models and with the first 10 principal components calculated from the above analysis used as covariates. Results from the GWAS were plotted using standard plotting functions in the R statistical language to enable replicability and analysis of the underlying data. In the resultant Manhattan plot (see Figure 2), it was quite clear that a singular peak on chicken chromosome 5 could be identified and distinguished from potential random noise in the dataset. In the plot, statistical significance was adjusted based on a Bonferroni correction based on the number of markers used in the association testing (red line). Any point above this line was considered a statistically significant association that was unlikely to result from random testing. Given that there were multiple markers in the region that met this criteria, it was difficult to distinguish among any such marker that could have sole-linkage to the phenotype. Our current best estimate is that the causal variant resides within a 1 megabase region on chicken chromosome 5, between these coordinates: Chr5:11600901-12789133. The markers with the significant association are exclusive to the controls, where the presence of a heterozygous copy of the marker provides a protective effect. The analysis predicts that the heterozygote state reduces the chances of a carrier having the disease by up to 3 times compared to non-carriers. This means that the haplotype is associated with a protective effect.

### 1.3 Potential biological significance and current state of the finding

A gene neighborhood screen was conducted in the identified region on chromosome 5. First, the coordinates were used to identify all annotated Refseq genes within the significant region (see Figure 3) and each gene was screened against the literature to identify potential disease impacts. Significant keywords included terms involving the liver or vaccination related diseases. Two candidate genes were identified in this first pass: GTF2H1 and TSG101. The General Transcription Factor IIH Subunit 1 (GTF2H1) gene is translated into a 61 kilodalton subunit (p61) of the essential transcription factor, TFIIH, that is associated with diseases such as Rift Valley Fever and Hermansky-Pudlak Syndrome 5. TSG101 (Tumor Susceptibility 101) is a signaling protein associated with diseases such as Hepatitis E and Charcot-Marie-Tooth Disease, Axonal, Type 2P. Both genes are on the 5' end of the identified significance region. Current evidence does not point to either gene as having a causal allele for the protective effect observed in the association analysis, but this has not been confirmed via more sensitive screening. Further testing is needed to identify the true causal variant within the region.

Rapid identification of carrier individuals can be conducted by using a haplotype test using one or more SNP markers from a set of eight non-consecutive SNP markers. The names of the markers, their coordinates, and the significance of their association are listed in Table 1.3 below. Genomic coordinates are based on the sequence of the galGal4 reference genome assembly (https://www.ncbi.nlm.nih.qov/assembly/GCF 000002315.3/). Publicly available markers are denoted by their NCBI dbSNP accession identifiers (rsid). The haplotype test can be conducted using prior state of the art SNPs using open-source available tools such as Flmpute. However, the accuracy of the haplotype will improve using the novel SNP markers provided herein that can provide better tracking of the segment.

**Table 1.3. Non-consecutive SNP markers comprised in the haplotype that protects against the post-vaccine hepatopathy, their coordinates and the significance of their association with the haplotype.**

| Marker Name | Chromosome | Position | p-value of association | Effect Allele | Other Allele | Status |
|---|---|---|---|---|---|---|
| HGC020997 | 5 | 11706079 | 6.15E-15 | A | C | |
| rs13757061 | 5 | 11821692 | 3.89E-15 | G | A | public |
| HGC021007 | 5 | 11903264 | 2.75E-16 | C | A | |
| HGC021012 | 5 | 12012528 | 1.70E-16 | G | A | |
| rs13757293 | 5 | 12224278 | 9.69E-14 | A | C | public |
| HGC021024 | 5 | 12365008 | 9.69E-14 | A | G | |
| rs15666151 | 5 | 12417674 | 9.69E-14 | A | C | public |
| HGC021027 | 5 | 12442360 | 9.69E-14 | G | A | |

### 1.4 Identification of causal variant

For identification of the exact causal variant behind the association, large-scale DNA sequencing analysis is performed, including up to 100 sequenced individuals that will consist of 50 cases and 50 controls. Functional genomic variant analysis is conducted as previously described (Ortega et al. 2022 J. Dairy Sci. 105, 9001-9011) with concordance testing to identify potential causal variants. Identification of the functional variant is used to design a test to directly rapidly identify carriers of the functional variant, which test can replace the haplotype test as the preferred method to identify the carrier state of affected birds.

## Claims

1. A method of testing an avian subject for a predisposition for having a reduced risk of developing post-vaccination hepatopathy, the method comprising determining in a sample comprising nucleic acids of the subject the presence of a haplotype associated with the predisposition, wherein the haplotype comprises at least one SNP selected from the group consisting of:
- A on position 11706079 of chromosome 5 of the galGal4 reference chicken genome assembly;
- G on position 11821692 of chromosome 5 of the galGal4 reference chicken genome assembly;
- C on position 11903264 of chromosome 5 of the galGal4 reference chicken genome assembly;
- G on position 12012528 of chromosome 5 of the galGal4 reference chicken genome assembly;
- A on position 12224278 of chromosome 5 of the galGal4 reference chicken genome assembly;
- A on position 12365008 of chromosome 5 of the galGal4 reference chicken genome assembly;
- A on position 12417674 of chromosome 5 of the galGal4 reference chicken genome assembly; and,
- G on position 12442360 of chromosome 5 of the galGal4 reference chicken genome assembly.

2. A method according to claim 1, wherein the presence of the haplotype is determined by detecting in the nucleic acid sample at least one SNP selected from the group consisting of:
- A on position 11706079 of chromosome 5 of the galGal4 reference chicken genome assembly;
- G on position 11821692 of chromosome 5 of the galGal4 reference chicken genome assembly;
- C on position 11903264 of chromosome 5 of the galGal4 reference chicken genome assembly;
- G on position 12012528 of chromosome 5 of the galGal4 reference chicken genome assembly;
- A on position 12224278 of chromosome 5 of the galGal4 reference chicken genome assembly;
- A on position 12365008 of chromosome 5 of the galGal4 reference chicken genome assembly;
- A on position 12417674 of chromosome 5 of the galGal4 reference chicken genome assembly; and,
- G on position 12442360 of chromosome 5 of the galGal4 reference chicken genome assembly.

3. A method according to claim 1 or 2, wherein the avian subject of the genus *Gallus,* preferably of the species *Gallus gallus domesticus.*

4. A method for selective breeding of avian subjects for a predisposition for having a reduced risk of developing post-vaccination hepatopathy, the method comprising:
a) determining in a sample comprising nucleic acids of the subject the presence of a haplotype as defined in claim 1, wherein preferably the presence of the haplotype is determined by detecting in the nucleic acid sample at least one SNP selected from the group consisting of:
- A on position 11706079 of chromosome 5 of the galGal4 reference chicken genome assembly;
- G on position 11821692 of chromosome 5 of the galGal4 reference chicken genome assembly;
- C on position 11903264 of chromosome 5 of the galGal4 reference chicken genome assembly;
- G on position 12012528 of chromosome 5 of the galGal4 reference chicken genome assembly;
- A on position 12224278 of chromosome 5 of the galGal4 reference chicken genome assembly;
- A on position 12365008 of chromosome 5 of the galGal4 reference chicken genome assembly;
- A on position 12417674 of chromosome 5 of the galGal4 reference chicken genome assembly; and,
- G on position 12442360 of chromosome 5 of the galGal4 reference chicken genome assembly, and,
b) removing a subject as breeding stock when the haplotype is absent and selecting a subject as breeding stock when the haplotype is present.

5. A method according to claim 4, wherein an avian subject that is selected as breeding stock is homozygous for the haplotype.

6. A method according to claim 5, wherein the avian subject of the genus *Gallus,* preferably of the species *Gallus gallus domesticus.*

7. Use of at least one SNP as defined in claim 1 for identification of a haplotype that is indicative of an avian subject subject's predisposition for having a reduced risk of developing post-vaccination hepatopathy.

8. A use according to claim 7, wherein the avian subject of the genus *Gallus,* preferably of the species *Gallus gallus domesticus.*

9. An isolated nucleic acid molecule or construct comprising a haplotype or a part or variant thereof, that is indicative of an avian subject subject's predisposition for having a reduced risk of developing post-vaccination hepatopathy, as comprised in the genome of Shaver White chicken, located on chromosome 5 within the chromosomal region delimited by:
- position 11706079 of chromosome 5 of the galGal4 reference chicken genome assembly; and,
- position 12442360 of chromosome 5 of the galGal4 reference chicken genome assembly,
wherein the nucleic acid molecule or construct, preferably comprises at least one SNP selected from the group consisting of:
- A on position 11706079 of chromosome 5 of the galGal4 reference chicken genome assembly;
- G on position 11821692 of chromosome 5 of the galGal4 reference chicken genome assembly;
- C on position 11903264 of chromosome 5 of the galGal4 reference chicken genome assembly;
- G on position 12012528 of chromosome 5 of the galGal4 reference chicken genome assembly;
- A on position 12224278 of chromosome 5 of the galGal4 reference chicken genome assembly;
- A on position 12365008 of chromosome 5 of the galGal4 reference chicken genome assembly;
- A on position 12417674 of chromosome 5 of the galGal4 reference chicken genome assembly; and,
- G on position 12442360 of chromosome 5 of the galGal4 reference chicken genome assembly.

10. An isolated nucleic acid molecule or construct according to claim 9, wherein the molecule or construct comprises a variant of the haplotype or a part thereof that has at least 60% nucleotide sequence identity with the haplotype as comprised in the genome of Shaver White chicken.

11. A marker for identification of a haplotype, wherein the haplotype, when present in the genome of an avian subject, is indicative of the subject's predisposition for having a reduced risk of developing post-vaccination hepatopathy, wherein the marker is a SNP selected from the group consisting of:
- A on position 11706079 of chromosome 5 of the galGal4 reference chicken genome assembly;
- C on position 11903264 of chromosome 5 of the galGal4 reference chicken genome assembly;
- G on position 12012528 of chromosome 5 of the galGal4 reference chicken genome assembly;
- A on position 12365008 of chromosome 5 of the galGal4 reference chicken genome assembly; and,
- G on position 12442360 of chromosome 5 of the galGal4 reference chicken genome assembly.

12. A marker according to claim 11, wherein the avian subject of the genus *Gallus,* preferably of the species *Gallus gallus domesticus.*

13. An oligonucleotide probe or primer comprising a SNP selected from the group consisting of:
- A on position 11706079 of chromosome 5 of the galGal4 reference chicken genome assembly;
- C on position 11706079 of chromosome 5 of the galGal4 reference chicken genome assembly;
- C on position 11903264 of chromosome 5 of the galGal4 reference chicken genome assembly;
- A on position 11903264 of chromosome 5 of the galGal4 reference chicken genome assembly;
- G on position 12012528 of chromosome 5 of the galGal4 reference chicken genome assembly;
- A on position 12012528 of chromosome 5 of the galGal4 reference chicken genome assembly;
- A on position 12365008 of chromosome 5 of the galGal4 reference chicken genome assembly;
- G on position 12365008 of chromosome 5 of the galGal4 reference chicken genome assembly;
- G on position 12442360 of chromosome 5 of the galGal4 reference chicken genome assembly; and,
- A on position 12442360 of chromosome 5 of the galGal4 reference chicken genome assembly
or an oligonucleotide probe or primer comprising a genomic sequence immediately adjacent of the SNP.

14. An array comprising at least one nucleic acid probe as defined in claim 13.

15. A kit comprising at least one nucleic acid probe or primer as defined in claim 13 or at least one array as defined in claim 14, and optionally at least another component for detecting in a sample comprising nucleic acids of an avian subject the presence of a haplotype as defined in claim 1.
